# EUROPÄISCHE PATENTSCHRIFT

(11) **EP 1 878 467 B1**
(45) Veröffentlichungstag und Bekanntmachung des Hinweises auf die Patenterteilung: **01.10.2008**
(21) Anmeldenummer: 07009889.2
(22) Anmeldetag: 18.05.2007
(51) Int. Cl.: A61N 5/06

(54) **Vertikales Besonnungsgerät mit verbesserter Kühl- und Frischluftzufuhr**
Vertical solarium device with improved fresh air supply
Appareil d'ensoleillement vertical avec alimentation d'air frais améliorée

(30) Priorität: 15.07.2006 DE 202006010969 U
(43) Veröffentlichungstag der Anmeldung: 16.01.2008
(73) Patentinhaber: KBL Solarien AG, 56307 Dernbach (DE)
(72) Erfinder: Lahr, Bernd, 56307 Dernbach (DE)
(74) Vertreter: Beyer, Rudi

(56) Entgegenhaltungen:
- DE-A1- 3 013 081
- DE-A1- 3 600 969
- DE-U1- 9 312 888
- US-A- 4 469 102
- US-A1- 2005 065 578

## Beschreibung

### Gattung

Die Erfindung betrifft ein vertikales Besonnungsgerät, mit einer um vertikale Drehachsen zu öffnenden und zu schließenden Tür, die mit feststehenden Wänden des Besonnungsgeräts einen Innenraum begrenzt, wobei in dem Innenraum eine Person aufrecht stehend sich durch die in den feststehenden Wänden angeordneten Besonnungslampen besonnen kann, mit wenigstens einem motorisch angetriebenen Lüfter zum Bewegen von Kühl- und Frischluft, die die Besonnungslampen und die Person in dem Innenraum beaufschlagt, wobei der Innenraum oben im Kopfbereich wenigstens eine Einströmöffnung zum Ansaugen von Kühl- und Frischluft aufweist, die im Bereich eines Standfußes durch wenigstens eine Abluftöffnung den Innenraum verlässt und durch in den Wänden des feststehenden Teils vorgesehenen Räumen nach oben strömt und die Besonnungslampen kühlt.

### Stand der Technik

Vertikale Besonnungsgeräte sind in einer großen Vielzahl unterschiedlicher Konstruktionen vorbekannt. Zahlreiche der vorbekannten vertikalen Besonnungsgeräte bestehen aus einer säulenartigen, aufrecht stehenden Besonnungskabine, die zum Beispiel an ihrer Außen- und Innenseite zylinderförmig gestaltet ist, wobei die um vertikale Drehachsen zu öffnende und zu schließende Tür einen Teil der den Innenraum begrenzenden Wandungen bildet. Durch die von der Tür zu verschließende Öffnung betritt die zu besonnende Person den Innenraum. Die Besonnungslampen können mit ihren Längsachsen parallel und mit Abstand zueinander angeordnete Röhren sein, die in den feststehenden Wandungen und in der Tür angeordnet sind und an eine Stromquelle über elektrische bzw. elektronische Bauteile anschließbar sind, um die Besonnungslampen mit Energie zu versorgen. Bei zahlreichen dieser vertikalen Besonnungsgeräte befindet sich im Kopfbereich ein motorisch angetriebener Axiallüfter, der die im Innenraum befindliche Luft aus diesem heraussaugt und nach außen befördert. Dies wird von zahlreichen Benutzern wegen des hohen Lärms im Kopfbereich als unangenehm empfunden. Außerdem ist besonders bei kalter Witterung die Gefahr von Erkältungen gegeben, da durch den zum Kühlen der Besonnungslampen erforderlichen Luftstrom die Person, besonders bei niedrigen Außentemperaturen, im Innenraum des Besonnungsgerätes friert.

Bekannt ist es auch, Frischluft zum Kühlen des Innenraumes und der Beleuchtungsröhren von unten durch ein Gebläse einzublasen und im oberen Bereich des Besonnungsgerätes wieder abzuführen. Dabei hat man auch schon vorgeschlagen, den einzelnen Wandungsteilen des vertikalen Besonnungsgerätes, sogenannten Elementen, jeweils eine eigene Belüftung, zum Beispiel eigene kleinere motorisch angetriebene Axiallüfter zuzuordnen, die die Besonnungslampen mit Kühlluft beaufschlagen. Dies ist konstruktionsbedingt aufwendig.

Aus der DE 93 12 888 U1 ist ein vertikales Besonnungsgerät gemäß dem Oberbegriff des Patentanspruches 1 vorbekannt, mit einer Anzahl von Bestrahlungsröhren, die entlang einer einen Bestrahlungsraum bildenden, strahlendämmenden und/oder reflektierenden Seitenwandung aneinandergereiht sind, in der ein strahlendämmend und/oder reflektierend verschließbarer Eingang vorgesehen ist. Dieses Besonnungsgerät weist einen Eingang auf, der unmittelbar in einen mit dem Bestrahlungsraum verbundenen Vorraum führt, der zusätzlich zum Bestrahlungsraum von der Seitenwandung ausgebildet und zusammen mit dem Bestrahlungsraum durch ein an der Seitenwand abgestütztes Deckenelement abgedeckt ist, wodurch eine integrierte, in sich nahezu geschlossene Zelle geschaffen wird.

Die US-A 4,469,102 zeigt ebenfalls ein Besonnungsgerät mit hexagonalem Querschnitt und mit im Deckenbereich angeordnetem Lüfter zur Kühlluftförderung. Die gekühlte Luft wird vom Boden aus durch die sandwichsartig aufgebauten Seitenwände geleitet.

Die DE 36 00 969 A1 beschreibt ein im Querschnitt dreieckförmig gestaltetes vertikales Besonnungsgerät mit einem mittig angeordneten Drehteller, auf dem die zu besonnende Person steht und an den Besonnungsgeräten vorbeigedreht wird. Im Kopfbereich ist ein Lüfter angeordnet, um die erhitzte Luft aus dem Innenraum abzusaugen und wegzufördern.

Die US 2005/0065578 A1 beschreibt ebenfalls ein vertikales Besonnungsgerät mit im wesentlichen hexagonalen Querschnitt und einem Lüfter im Kopfbereich des Gerätes, um Kühlluft zu fördern, die durch Schlitze geführt wird.

### Aufgabe

Der Erfindung liegt die Aufgabe zugrunde, ein vertikales Besonnungsgerät derart auszugestalten, dass eine verbesserte Kühlluft- und Frischluftzufuhr sowohl für die Besonnungslampen als auch für die zu besonnende Person erreicht wird, bei zweckmäßiger Konstruktion.

### Lösung

Die der Erfindung zugrunde liegende Aufgabe wird durch die in **Patentanspruch 1** wiedergegebenen Merkmale gelöst.

### Einige Vorteile

Bei dem erfindungsgemäßen vertikalen Besonnungsgerät ist der Innenraum oben im Kopfbereich offen ausgebildet, das heißt, hier befindet sich kein Gebläse, sondern mindestens eine, vorzugsweise nur eine, nach außen in die Umgebung ausmündende Öffnung großen Querschnitts, durch die Frisch- und Kühlluft in den Innenraum eintreten kann. Die Luft wird durch diese Öffnung in den Innenraum z. B. eingesogen, umspült hierbei die im Innenraum befindliche zu besonnende Person und verlässt dann den Innenraum durch mindestens eine im Bodenbereich des Innenraums befindliche Öffnung, strömt außerhalb des Innenraums wieder nach oben, umspült und kühlt die Beleuchtungslampen, wird im oberen Bereich, also oberhalb des Kopfbereiches der zu besonnenden Person in einem gemeinsamen Ringkanal gesammelt und durch einen an den Ringkanal angeschlossenen Luftabführkanal oder eine hier vorgesehene gemeinsame Öffnung wieder abgeführt. Das Abführen der Kühlluft kann entweder in den Umgebungsraum, oder aber über einen Kanal, Schlauch oder Leitung nach außen, zum Beispiel in die freie Atmosphäre außerhalb des Aufstellungsraumes für das vertikale Besonnungsgerät, geschehen. Auf diese Weise tritt die Kühlluft in den Innenraum durch praktisch ungestörte Axialströmung in diesen ein, umspült den Körper der zu besonnenden Person, verlässt den Innenraum durch wenigstens eine, vorzugsweise durch mehrere Öffnungen im Fußbereich des Innenraums und tritt in einen oder in mehrere Kanäle ein, um in die Räume zu strömen, in denen sich die Besonnungslampen befinden. Das bedeutet, dass auch die der Tür zugeordneten Besonnungslampen von der im Fußbereich abströmenden Kühlluft beaufschlagt und gekühlt werden.

Dadurch, dass im Kopfbereich vorzugsweise nur eine einzige, den Querschnitt des Innenraumes aufweisende zentrische Einströmöffnung angeordnet ist, kann eine relativ große Luftmenge über ein außerhalb dieser Einströmöffnung, zum Beispiel an der der Einströmöffnung abgekehrten äußeren Peripherie der Ringsammelleitung befindliches Gebläse gefördert werden, so dass einerseits die Frischluft ungestört in die relativ große Einströmöffnung einströmen und den Körper der zu besonnenden Person von oben kommend umspülen kann, während andererseits das Geräusche verursachende Gebläse weg vom Kopfbereich an der äußeren Peripherie der Ringsammelleitung anzuordnen ist und nicht mehr unmittelbar oberhalb des Kopfes wie es bei Axiallüftern des Standes der Technik der Fall ist, angeordnet ist. Dadurch wird einerseits das Umströmen des Körpers als angenehmer empfunden, andererseits ist die Geräuschquelle vom Kopf der zu besonnenden Person weiter entfernt angeordnet. Durch die relativ große zentrische Einströmöffnung hat die Person auch nicht das Gefühl des Eingesperrtseins.

Durch diese Konstruktion hat man es in im übrigen in der Hand, den Antriebsmotor des vorzugsweise als Radialgebläse ausgebildeten Gebläses für das Ansaugen der Luft so zu gestalten, dass mit minimalem Luftverbrauch gearbeitet werden kann. Dieser Luftverbrauch kann einerseits auf die Temperatur der zu kühlenden Besonnungslampen, andererseits aber im Bedarfsfalle auch auf die Außentemperatur der Luft durch geeignete Temperaturfühler abgestimmt sein. Dies kann zum Beispiel bei kalter Jahreszeit dazu führen, dass die zu besonnende Person den Innenraum betritt, die Tür schließt und die Besonnungslampen einschaltet, wobei am Anfang und je nach Außentemperatur unter Umständen der Antriebsmotor des Gebläses noch gar nicht anspringt. Dies führt wiederum dazu, dass die Person nicht mit unangenehm kalter Kühlluft beaufschlagt wird, sondern die Besonnungslampen zunächst ihre Tätigkeit aufnehmen, wobei durch den Infrarotanteil der sich im Verlaufe der Besonnungszeit erhitzende Körper durch den nach einiger Zeit anlaufenden Lüfter allmählich und sanft gekühlt und nicht von Anfang an mit einer großen Kühlluftmenge beaufschlagt wird.

Dadurch, dass die Drehzahl des Antriebsmotors für den vorzugsweise als Radialgebläse ausgebildeten Gebläses steuer- oder regelbar ist, und zwar vorzugsweise in Abhängigkeit der jeweiligen Temperatur der zu kühlenden Besonnungslampen einerseits und z. B. der Außentemperatur andererseits, hat man es in der Hand, jeweils mit der optimalen geringsten Kühlluftmenge zu arbeiten.

### Weitere erfinderische Ausgestaltungen

Weitere erfinderische Ausgestaltungen sind in den **Patentansprüchen 2** bis **17** beschrieben.

Bei der Ausführungsform nach **Patentanspruch 2** ist die Einströmöffnung zentrisch angeordnet und entspricht vorzugsweise dem Innendurchmesser des Innenraums, wobei die Einströmöffnung axial und zum Beispiel auch radial durch einen oberen Abschlusskörper begrenzt ist, der einen gemeinsamen Ringsammelkanal aufweist, dem ein Gebläse an der dem Innenraum abgekehrten Seite seitlich zugeordnet ist.

Bei der Ausführungsform nach **Patentanspruch 3** ist das Gebläse ein Radialgebläse.

Gemäß **Patentanspruch 4** fördert das Gebläse über eine nach oben in den freien Raum ausmündende Anschlussöffnung die Abluft entweder in den umgebenden Raum oder über einen Anschlusskanal oder über einen Schlauch aus dem Raum, in dem das Besonnungsgerät angeordnet ist, heraus.

Die Ausführungsform nach **Patentanspruch 5** ist dadurch gekennzeichnet, dass der gemeinsame Ringsammelkanal an der oberen Stirnseite des feststehenden Teils des Besonnungsgerätes sämtliche der Kühlung der Besonnungslampen dienenden Räume miteinander kühlluftleitend verbindet.

Gemäß **Patentanspruch 6** ist dem Antriebsmotor des Gebläses eine Steuer-oder Regelvorrichtung zum Regeln oder Steuern der Drehzahl zugeordnet.

Die Ausführungsform nach **Patentanspruch 7** besitzt einen oder mehrere Temperaturfühler, die die Temperatur der Lampen, zum Beispiel an deren Oberfläche oder in unmittelbarer Nähe dazu und/oder die außerhalb des Besonnungsgerätes befindliche Kühlluft messen und in Abhängigkeit dieser Temperaturen die Drehzahl des Antriebsmotors für das Gebläse steuern oder regeln.

Bei der Ausführungsform nach **Patentanspruch 8** sind eine oder mehrere Sonden dem Besonnungsgerät z. B. an seiner Außenseite zugeordnet, die die Außenlufttemperatur in dem Aufstellungsraum für das Besonnungsgerät messen, während eine oder mehrere Sonden die Hauttemperatur der zu besonnenden Person messen, wobei die Werte an einen Speicher mit hinterlegten Daten eines Zentralrechners für eine Steuer- oder Regelanlage zum Steuern oder Regeln der Drehzahl des Antriebsmotors für das Gebläse weitergeben wird und in Abhängigkeit dieser Temperaturen die Drehzahl des Antriebsmotors und damit auch die Luftmenge, die jeweils in den Innenraum des Besonnungsgerätes angesaugt wird, gesteuert oder geregelt wird.

Bei der Ausführungsform nach **Patentanspruch 9** wird in Abhängigkeit der Leistungsaufnahme der jeweiligen Besonnungslampen die Drehzahl des Antriebsmotors gesteuert oder geregelt.

Gemäß **Patentanspruch 10** ist der gemeinsame Ringsammelkanal kreisringförmig ausgebildet und begrenzt die obere ringförmige Wand des Besonnungsgerätes.

Gemäß **Patentanspruch 11** sind die Wandungen des gemeinsamen Ringsammelkanals abgerundet bzw. so gestaltet, dass sie der äußeren Kontur der Außenseite des Besonnungsgerätes formmäßig angepasst sind.

**Patentanspruch 12** beschreibt eine vorteilhafte Ausführungsform der Erfindung. Vorteilhafterweise ist der innere (lichte) Querschnitt der Abluftleitung kreisringförmig, polygonförmig oder rechteckförmig gestaltet.

Gemäß **Patentanspruch 13** ist im Fußbereich des Innenraumes eine schlitzförmige Abluftöffnung vorgesehen, die in Kanäle ausmünden, durch die die Kühlluft in die Räume eintritt, in denen sich die jeweiligen Besonnungslampen befinden.

Bei der Ausführungsform gemäß **Patentanspruch 14** sind die mit ihren Längsachsen parallel zueinander sowie mit Abstand zueinander um die Längsmittenachse des Innenraumes angeordneten Besonnungslampen in einem Raum oder in mehreren Räumen angeordnet, in denen durch die im Fußbereich angeordnete Schlitze Kühlluft einströmt.

Bei der Ausführungsform nach **Patentanspruch 15** ist das Gebläse über einen Ansaugstutzen mit einem Abschlusskörper verbunden, der den Ringsammelkanal aufweist. Die Schalldämmung kann zum Beispiel aus den Silentblöcken oder sonstigen schwingungsdämpfenden Elementen bestehen, um die Übertragung des Körperschalls von dem Gebläse auf die Wände des Besonnungsgerätes weitgehend zu mindern. **Patentanspruch 16** beschreibt eine vorteilhafte und stabile Ausführungsform.

Bei der Ausführungsform nach **Patentanspruch 17** sind die Besonnungslampen in Kreissektorzonen aufgeteilt.

In der Zeichnung ist die Erfindung - teils schematisch - beispielsweise veranschaulicht. Es zeigen:
- Fig. 1: ein vertikales Besonnungsgerät in perspektivischer Darstellung bei geöffneter Tür und
- Fig. 2: das aus Fig. 1 ersichtliche Gerät mit eingezeichneten Luftströmungspfeilen und sichtbar gemachtem, als Radialgebläse ausgebildeten Gebläse, bei abgenommener Tür.

Mit 1 ist insgesamt ein vertikales Besonnungsgerät bezeichnet, das an seiner Außenseite 2 zylindrisch gestaltet ist. Auch der von der zu besonnenden Person zu betretende Innenraum 3 ist durch eine zylindrische Innenwand 4 umgrenzt, die teilweise durch die Innenseite einer zu öffnenden Tür 5 gebildet wird, die auch einen Teil der zylindrischen Außenseite 2 darstellt.

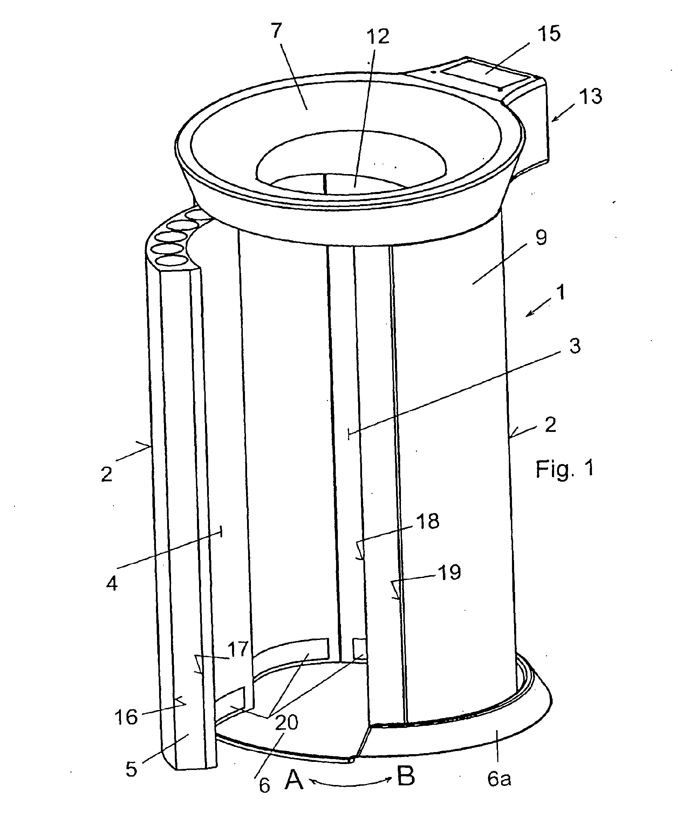

Die Tür 5 ist über nicht dargestellte, vertikal verlaufende Achsen in horizontaler Ebene, also in Richtung A bzw. B (Fig. 1) schwenkbeweglich ausgebildet und damit zu öffnen und z. B. spaltdicht zu schließen. Nach dem Schließen der Tür 5 bildet die Tür 5 mit dem übrigen festen Bestandteil des vertikalen Besonnungsgerätes 1 einen überwiegend innen und außen zylindrischen Körper, der unten einen Standfuß 6 aufweist, der durch eine ebene Platte gebildet ist, auf der die zu besonnende Person im Innenraum 3 steht und der zum Beispiel im Innenraum 3 mit einem hautfreundlichen Gewebe, einer abwaschbaren oder zu desinfizierenden Matte oder dergleichen ausgestattet sein kann. Der Standfuß 6 weist einen Kragen 6a auf, der gegenüber der als zylindrische Außenmantelfläche ausgebildeten Außenseite 2 nach oben etwas überkragt.

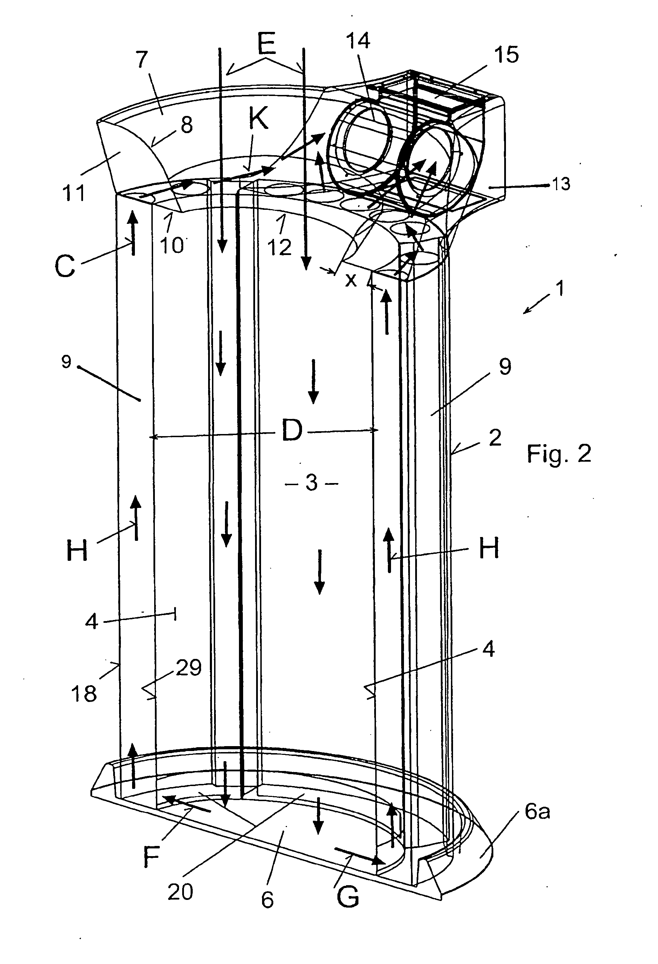

Oben wird das vertikale Besonnungsgerät 1 durch einen oberen Abschlusskörper 7 begrenzt, der im Querschnitt (Fig. 2) eine etwa trapezförmige Gestalt aufweist, wobei eine der parallel zueinander verlaufenden Seiten abgerundet ausgebildet ist und in einen relativ flachen Kreisbogen 8 in die untere auf den zylindrischen Teil 9 des vertikalen Besonnungsgerätes 1 aufliegenden Basisseite 10 übergeht. Im übrigen ist der obere Abschlusskörper 7 in der Draufsicht kreisringförmig ausgestaltet und bildet einen gemeinsamen Ringsammelkanal 11 für die in Richtung C abströmende Kühlluft.

Der obere Abschlusskörper 7 weist eine zentrale in der Draufsicht ringförmige Einströmöffnung 12 auf, die in der Draufsicht kreisförmig gestaltet ist und deren Durchmesser bei der dargestellten Ausführungsform etwas geringer als der Innendurchmesser D des Innenraumes 3 bemessen ist. Der Durchmesser der Einströmöffnung 12 ist um den auf diametral gegenüberliegenden Seiten in den Innenraum hervorspringenden Kreisringanteil zwei Mal X verringert. Es ist aber auch möglich, den Durchmesser der Einströmöffnung 12 gleich dem Durchmesser D des Innenraumes 3 zu gestalten.

Mit dem oberen Abschlusskörper 7 ist ein kastenförmiger Ansatz 13 einstückig verbunden, in dem ein bei der dargestellten Ausführungsform als Radialgebläse ausgebildetes Gebläse 14 angeordnet ist. Das Radialgebläse 14 wird durch einen im Einzelnen nicht dargestellten Elektromotor mit regelbarer Drehzahl angetrieben, dem Energie durch eine ebenfalls nicht dargestellte elektrische Leitung von einer geeigneten elektrischen Quelle zugeführt wird. Auch die Regel- oder Steueranlagen für den Antriebsmotor des Gebläses 14 sind aus Gründen der Vereinfachung der Zeichnung im Einzelnen nicht dargestellt. Der kastenförmige Ansatz 13 besitzt an seiner aus Fig. 2 ersichtlichen Oberseite eine Anschlussöffnung 15, an die ein Abluftkanal angeschlossen ist. Dieser Abluftkanal kann durch einen Teil des kastenförmigen Ansatzes 13 selbst gebildet sein, so dass die durch die Anschlussöffnung 15 austretende Abluft sofort in die Umgebung austritt. Es ist aber auch möglich, an diese Anschlussöffnung 15 ein Rohr, einen Schlauch oder Kanal anzuschließen, durch den die Luft entweder in weiterer Entfernung in den Raum geleitet oder nach außen aus dem Gebäude herausgeleitet wird. Im übrigen kann der kastenförmige Ansatz 13 über schwingungsdämpfende Lager über Flansche (nicht dargestellt) mit dem oberen Anschlusskörper 7 verbunden sein. Zusätzlich oder stattdessen können auch die Lager für die Motorwelle in schwingungsdämpfende Lager angeordnet sein, um den Körperschall, der sich auf den zylindrischen Teil 9 und damit auch auf den Innenraum 3 übertragen könnte, zu mindern.

Sowohl die Tür 5 als auch der stationäre zylindrische Teil 9 des vertikalen Besonnungsgerätes können durch sandwichartig zueinander geordnete Wände 16, 17 bzw. 18, 19 begrenzt sein. Innerhalb der so gebildeten Ringräume ist (nicht dargestellt) jeweils eine Mehrzahl oder Vielzahl von mit Abstand sowie mit ihren Längsachsen parallel zueinander angeordneten, als Röhren ausgebildete Beleuchtungslampen angeordnet, deren elektrische bzw. elektronische Steuer- und Regeleinrichtungen ebenfalls nicht dargestellt sind. Zwischen den so in den ringförmigen Räumen der Tür 5 und des zylindrischen Teils 9 angeordneten Beleuchtungslampen sind Zwischenräume vorhanden, durch die Kühlluft hindurchtreten und die Beleuchtungslampen entsprechend kühlen kann. Z. B. sind in der Tür 5 und in dem Teil 9 insgesamt 52 Beleuchtungslampen vorgesehen.

Unten im Bereich des Standfußes 6 sind im Innenraum 3 mehrere schlitzförmige Abluftöffnungen 20 angeordnet, durch die die Kühlluft in die durch die Wände 16, 17 bzw. 18, 19 begrenzenden Zwischenräume eintritt und dadurch die dort angeordneten Besonnungslampen kühlt.

Die Wirkungsweise des aus der Zeichnung ersichtlichen vertikalen Besonnungsgerätes ist folgende:

Angenommen, der Antriebsmotor des Radialgebläses 14 wird angetrieben, dann wird in Richtung E Luft durch die Einströmöffnung 12 angesaugt, die dadurch in den Innenraum 3 eintritt und auch den Körper der zu besonnenden Person umspült. Die Kühlluft verlässt den Innenraum 3 durch die Abluftöffnungen 20 im Bereich des Standfußes 6 gemäß den Pfeilen F und G (Fig. 2) und strömt durch die durch die Wände 16, 17 bzw. 18, 19 gebildeten Hohlräume in Richtung H und C nach oben und tritt über entsprechende Anschlussöffnungen in den Ringsammelkanal 11 ein, von wo aus die Abluft gesammelt wird und in Richtung K zu dem Radialgebläse 14 strömt, das die Abluft durch die Anschlussöffnung 15 in einen Abluftkanal, Abluftschlauch oder in die unmittelbare Umgebung wegfördert.

Der Antriebsmotor des Gebläses 14 wird in seiner Drehzahl und damit in seiner Förderleistung entsprechend der Temperatur der in Richtung E strömenden Kühlluft und/oder der im Innenraum 3 gemessenen Temperatur geregelt oder gesteuert. Ist die Temperatur der so angesaugten Luft gering, wird der Antriebsmotor des Gebläses 14 gar nicht oder nur mit geringer Drehzahl angetrieben. Erwärmt sich die Luft in dem Innenraum 3, wird die Antriebsleistung bzw. die Drehzahl vergrößert, was auch eine entsprechend größere angesaugte Luftmenge zur Folge hat.

Stattdessen oder zusätzlich kann aber auch die Temperatur der nicht dargestellten Besonnungslampen unmittelbar, oder deren Besonnungsleistung oder der Raumtemperatur gemessen werden, die in dem Raum herrscht, in dem sich die Besonnungslampen befinden, also innerhalb der jeweiligen Ringräume, die durch die Wände 16, 17 bzw. 18, 19 begrenzt werden. Es ist auch möglich, die Antriebsleistung und damit die Drehzahl des Antriebsmotors für das Gebläse 14 ausschließlich nach der in diesen Ringräumen gemessenen Temperatur oder der Besonnungsleistung der Besonnungslampen zu steuern oder zu regeln.

Zusätzlich oder statt dessen bei allen Steuerungen und Regelungen kann außerdem die Hauttemperatur an einer oder an mehreren besonders empfindlichen Stellen der zu besonnenden Person durch nicht dargestellte Sensoren gemessen werden, wobei in einem Speicher einer Recheneinheit ein Programm hinterlegt werden kann, das empirische Daten aufweist, mittels derer die jeweils gemessenen Temperaturen der zu besonnenden Person verglichen werden, woraufhin dann die Drehzahl des Gebläses 14 entsprechend gesteuert bzw. geregelt wird. Angestrebt wird dabei, mit möglichst geringer Förderleistung und damit für den jeweiligen Anwendungsfall möglichst geringen Luftmenge zu arbeiten, damit im Winter die den Innenraum 3 betretende Person durch die durch die Einströmöffnung 12 angesaugte Luft nicht friert, andererseits aber auch ein Überhitzen der Besonnungslampen verhindert wird.

Wie man sieht, ist außerdem das Gebläse 14 mit seinem Antriebsmotor außerhalb des Kopfbereiches an der äußeren Peripherie des vertikalen Besonnungsgerätes 1 angeordnet, so dass auch die Schallquelle aus dem unmittelbaren Kopfbereich möglichst weit entfernt worden ist.

### Bezugszeichen

- 1: Besonnungsgerät, vertikales
- 2: Außenseite, Mantelfläche, zylindrische
- 3: Innenraum
- 4: Innenwand, zylindrische
- 5: Tür
- 6: Standfuß
- 6a: Kragen
- 7: Abschlusskörper, oberer
- 8: Kreisbogen
- 9: Teil, zylindrischer
- 10: Basisseite
- 11: Ringsammelkanal, gemeinsamer
- 12: Einströmöffnung
- 13: Ansatz, kastenförmiger
- 14: Gebläse, Radialgebläse
- 15: Anschlussöffnung
- 16: Wand
- 17: ,,
- 18: ,,
- 19: ,,
- 20: Abluftöffnung

- A: Schwenkrichtung der Tür 5
- B: ,, ,, ,,
- C: Strömungsrichtung
- D: Innendurchmesser des Innenraumes 3
- E: Strömungsrichtung der Kühlluft
- F: ,, ,, ,,
- G: ,, ,, ,,
- H: ,, ,, ,,
- K: ,, ,, ,,
- X: ,, ,, ,,
- X: Kreisringanteil

### Literaturverzeichnis

Prospekt "Sun planet", Moskau, Russland
Prospekt "ISO ITALIA Wellness Project Master Catalogue", ISO ITALIA srl, 30029 S. Stino di Livenza VE, Italy
Prospekt "magic sun", Moskau, Russland
Prospekt "logicam group, low pressure", Logicam Group, 26855 Lodivecchio (LODI), Italy
Prospekt "logicam group, opensun", NetSol Catalana de Rayos UVA S.L., 08016 Barcelona, Spanien
Prospekt "Feel the Difference, Soltron The Sunmaker, Serie XL, Serie M, Serie S, Serie V", Soltron GmbH, 31073 Delligsen
Prospekt "Studiosupport, Professionelle Ideen rund um's Studio, Soltron The Sunmaker", Soltron GmbH, 31073 Delligsen

## Patentansprüche

1. Vertikales Besonnungsgerät (1), mit einer um vertikale Drehachsen zu öffnenden und zu schließenden Tür (5), die mit feststehenden Wänden (18, 19) des Besonnungsgeräts (1) einen Innenraum (3) begrenzt, wobei in dem Innenraum (3) eine Person aufrecht stehend sich durch die in den feststehenden Wänden (18, 19) angeordneten Besonnungslampen besonnen kann, mit wenigstens einem motorisch angetriebenen Lüfter (14) zum Bewegen von Kühl- und Frischluft, die die Besonnungslampen und die Person in dem Innenraum (3) beaufschlagt, wobei der Innenraum (3) oben im Kopfbereich wenigstens eine Einströmöffnung (12) zum Ansaugen (E) von Kühl- und Frischluft aufweist, die im Bereich eines Standfußes (6) durch wenigstens eine Abluftöffnung (20) den Innenraum (3) verlässt und durch in den Wänden (18, 19) des feststehenden Teils (9) vorgesehenen Räumen nach oben (H bzw. C) strömt und die Besonnungslampen kühlt, **dadurch gekennzeichnet, dass** die Tür (5) ebenfalls Besonnungslampen aufweist, die gekühlt werden von Kühl- und Frischluft, die durch in den Wänden (16, 17) der Tür (5) vorgesehenen Räumen nach oben (H bzw. C) strömt; und **dadurch**, **dass** die Kühl- und Frischluft das Besonnungsgerät (1) über einen im Kopfbereich angeordneten gemeinsamen Ringsammelkanal (11), der an die Außenluft oder über einen Abluftkanal angeschlossen ist, verlässt.

2. Vertikales Besonnungsgerät nach Anspruch 1, **dadurch gekennzeichnet, dass** der Innenraum (3) oben im Kopfbereich eine zentrische, vorzugsweise dem Innendurchmesser (D) des Innenraums (3) entsprechende oder kleiner bemessene Einströmöffnung (12) aufweist, wobei die Einströmöffnung (12) axial und z. B. auch radial durch einen oberen Abschlusskörper (7) begrenzt ist, der einen gemeinsamen Ringsammelkanal (11) aufweist, dem ein Gebläse (14) an der dem Innenraum (3) abgekehrten Seite seitlich zugeordnet ist.

3. Vertikales Besonnungsgerät nach Anspruch 1 oder 2, **dadurch gekennzeichnet, dass** der als Gebläse ausgebildete Lüfter (14) ein Radialgebläse ist.

4. Vertikales Besonnungsgerät nach Anspruch 1 oder einem der Ansprüche 2 und 3, **dadurch gekennzeichnet, dass** das Gebläse (14) über eine nach oben in den freien Raum ausmündende Anschlussöffnung (15) die Abluft entweder in den umgebenden Raum oder über einen Anschlusskanal oder über einen Schlauch aus dem Raum, in dem das Besonnungsgerät (1) angeordnet ist, herausbefördert.

5. Vertikales Besonnungsgerät nach Anspruch 1 oder einem der Ansprüche 2 bis 4, **dadurch gekennzeichnet, dass** der gemeinsame Ringsammelkanal (11) an der oberen Stirnseite des feststehenden Teils (9) des Besonnungsgerätes (1) sämtliche der Kühlung der Besonnungslampen dienenden Räume miteinander kühlluftleitend verbindet.

6. Vertikales Besonnungsgerät nach Anspruch 1 oder einem der Ansprüche 2 bis 5, **dadurch gekennzeichnet, dass** dem Antriebsmotor des Gebläses (14) eine Steuer- oder Regelvorrichtung zum Regeln oder Steuern der Drehzahl zugeordnet ist.

7. Vertikales Besonnungsgerät nach Anspruch 1 oder einem der Ansprüche 2 bis 6, **dadurch gekennzeichnet, dass** über einen oder über mehrere Temperaturfühler die Temperatur der Besonnungslampen, z. B. an deren Oberfläche oder in dem Raum, in dem sich die betreffenden Besonnungslampen befinden messbar ist, und/oder die außerhalb des Besonnungsgerätes (1) befindliche Kühllufttemperatur messbar ist und in Abhängigkeit dieser Temperaturen die Drehzahl des Antriebsmotors für das Gebläse (14) über eine elektronische Steuer- oder Regelvorrichtung steuer- oder regelbar ist.

8. Vertikales Besonnungsgerät nach Anspruch 7, **dadurch gekennzeichnet, dass** die in den Räumen, in denen sich die Besonnungslampen befinden gemessene Temperatur und die gemessene Temperatur der einströmenden (E) Kühlluft mit der durch wenigstens einen auf der Haut der zu besonnenden Person angeordneten Temperaturfühler messbar ist und diese Temperaturmesswerte in einen elektronischen Speicher eingebbar ist und hier mit abgespeicherten Werten vergleichbar und in Abhängigkeit von diesen Werten die Drehzahl des Antriebmotors und damit die Förderleistung des Gebläses (14) regel- oder steuerbar ist.

9. Vertikales Besonnungsgerät nach Anspruch 1 oder einem der Ansprüche 2 bis 8, **dadurch gekennzeichnet, dass** die Förderleistung des Gebläses (14) in Abhängigkeit der jeweiligen Leistungsaufnahme der Besonnungslampen steuer- oder regelbar ist.

10. Vertikales Besonnungsgerät nach Anspruch 1 oder einem der Ansprüche 2 bis 8, **dadurch gekennzeichnet, dass** der gemeinsame Ringsammelkanal (11) in der Draufsicht auf das Besonnungsgerät (1) kreisringförmig ausgebildet ist und die bei der geschlossenen Tür (5) obere z. B. ringförmige Wand des Besonnungsgerätes (1) begrenzt.

11. Vertikales Besonnungsgerät nach Anspruch 1 oder einem der Ansprüche 2 bis 10, **dadurch gekennzeichnet, dass** die Wandungen des gemeinsamen Ringsammelkanals (11) außen abgerundet bzw. so gestaltet sind, dass sie der äußeren Kontur der Außenseite (2) des Besonnungsgerätes (1) formmäßig angepasst sind.

12. Vertikales Besonnungsgerät nach Anspruch 1 oder einem der Ansprüche 2 bis 11, **dadurch gekennzeichnet, dass** das Gebläse (14) über eine im Querschnitt rechteckförmige quadratische Anschlussöffnung (15) die Abluft wegfördert und dass diese Anschlussöffnung (15) Teil eines kastenförmigen Ansatz (13) bildet, der mit dem gemeinsamen Ringsammelkanal (11) einstückig verbunden ist, dessen innerer Strömungsquerschnitt kreisförmig, oder polygonfömrig, oder rechteckförmig, oder quadratisch, gestaltet ist.

13. Vertikales Besonnungsgerät nach Anspruch 1 oder einem der Ansprüche 2 bis 12, **dadurch gekennzeichnet, dass** im Fußbereich des Innenraums (3) wenigstens eine schlitzförmige Abluftöffnung (20), vorzugsweise mehrere derartige schlitzförmige oder lang gestreckte Abluftöffnungen (20) mit Abstand sowie über die Peripherie des Innenraumes (3) verteilt mit gleichmäßigen Abständen zueinander angeordnet sind, durch die die Luft in die die Besonnungslampen aufnehmenden Räume der Tür (5) und des feststehenden Teils (9) des Besonnungsgerätes (1) einströmt.

14. Vertikales Besonnungsgerät nach Anspruch 1 oder einem der Ansprüche 2 bis 13, **dadurch gekennzeichnet, dass** in dem feststehenden Teil (9) und in der Tür (5) mit ihren Längsachsen parallel und mit Abstand zueinander Besonnungslampen angeordnet sind, deren sie aufnehmende Räume an den gemeinsamen Ringsammelkanal (11) und andererseits im Bereich eines Standfußes (6) an den Innenraum (3) über eine oder mehrere Abluftöffnungen (20) angeschlossen sind.

15. Vertikales Besonnungsgerät nach Anspruch 1 oder einem der Ansprüche 2 bis 14, **dadurch gekennzeichnet, dass** das Gebläse (14) über einen Ansaugstutzen, z. B. unter Zwischenschaltung eines schwingungsdämpfenden Lagers, an einen oberen, kreisringförmigen Abschlusskörper (7) angeschlossen sind, der in seinem Inneren den gemeinsamen Ringsammelkanal (11) aufweist.

16. Vertikales Besonnungsgerät nach Anspruch 15, **dadurch gekennzeichnet, dass** dem Anschlussstutzen für den kastenförmigen Ansatz (13) ein Schalldämmungskörper zugeordnet ist.

17. Vertikales Besonnungsgerät nach Anspruch 1 oder einem der Ansprüche 2 bis 16, **dadurch gekennzeichnet, dass** die Besonnungslampen in Kreissektoren zu mehreren Besonnungslampen zusammengefasst in den sandwichsartig aufgebauten Wänden (16, 17) der Tür (5) einerseits bzw. (18, 19) des feststehenden Teils (9) des Besonnungsgeräts (1) andererseits angeordnet sind und dass jeder Kreisringsektor über eine im Bereich des Standfußes (6) in den Innenraum (3) ausmündende schlitzförmige Abluftöffnung (20) an den jeweiligen Ringsektor angeschossen sind und dass die so gebildeten einzelnen Ringsektorkanäle an ihrer Oberseite in den gemeinsamen Ringsammelkanal (11) des oberen Abschlusskörpers (7) ausmünden.

## Claims

1. Upright sun-tanning booth (1), having a door (5) which opens and closes about vertical pivots and which together with fixed walls (18, 19) of the sun-tanning booth (1) delimits an interior space (3), where in the interior space (3) a person standing upright can be shone on by the sun-tanning lamps disposed in the fixed walls (18, 19), and having at least one motor-driven ventilator (14) for conveying cooling and fresh air which blows onto the sun-tanning lamps and the person in the interior space (3), where the interior space (3) incorporates above in the head area at least one inlet hole (12) for drawing in (E) cooling and fresh air, which in the area of a base (6) leaves the interior space (3) through at least one exhaust air hole (20) and flows upwards (H and C) through spaces provided in the walls (18, 19) of the fixed portion (9) and cools the sun-tanning lamps, **characterised in that** the door (5) likewise incorporates sun-tanning lamps which are cooled by cooling and fresh air, which flows upwards (H and C) through spaces provided in the walls (16, 17) of the door (5); **and in that** the cooling and fresh air leaves the sun-tanning booth (1) through a ring-shaped collector duct (11) disposed in the head area and connected to the outside air or an exhaust duct.

2. Upright sun-tanning booth in accordance with claim 1, **characterised in that** above in the head area the interior space (3) incorporates a centric inlet hole (12) of preferably the same size as the inside diameter (D) of the interior space (3) or smaller, where the inlet hole (12) is axially and e.g. also radially delimited by an upper closing member (7) which incorporates a common ring-shaped collector duct (11) to which a fan (14) is assigned at the side facing away from the interior space (3).

3. Upright sun-tanning booth in accordance with claim 1 or 2, **characterised in that** the ventilator (14) constructed as a fan is a radial fan.

4. Upright sun-tanning booth in accordance with claim 1 or any one of claims 2 and 3, **characterised in that** the fan (14) conveys the exhaust air through a connecting hole (15) leading upwards into the free space, either into the surrounding space or through an exhaust duct or a hose out of the space in which the sun-tanning booth (1) is disposed.

5. Upright sun-tanning booth in accordance with claim 1 or any one of claims 2 to 4, **characterised in that** the common ring-shaped collector duct (11) conducting cooling air connects all the spaces serving to cool the sun-tanning lamps on the upper front side of the fixed portion (9) of the sun-tanning booth (1).

6. Upright sun-tanning booth in accordance with claim 1 or any one of claims 2 to 5, **characterised in that** the drive motor of the fan (14) is assigned a control or regulator device to control or regulate the speed.

7. Upright sun-tanning booth in accordance with claim 1 or any one of claims 2 to 6, **characterised in that** the temperature of the sun-tanning lamps, e.g. on their surface or in the space in which these sun-tanning lamps are located, can be measured and/or the cooling air temperature outside the sun-tanning booth (1) can be measured by means of one or more temperature sensors and the speed of the drive motor for the fan (14) can be controlled or regulated according to these temperatures by means of an electronic control or regulating device.

8. Upright sun-tanning booth in accordance with claim 7, **characterised in that** the measured temperature in the spaces in which the sun-tanning lamps are located and the measured temperature of the inflowing (E) cooling air can be measured by means of at least one temperature sensor disposed on the skin of the person to be sun-tanned and these temperature measurements can be entered in an electronic memory and there compared with stored values and that the speed of the drive motor and thus the air output of the fan (14) can be controlled or regulated according to these values.

9. Upright sun-tanning booth in accordance with claim 1 or any one of claims 2 to 8, **characterised in that** the air output of the fan (14) can be controlled or regulated according to the respective power requirement of the sun-tanning lamps.

10. Upright sun-tanning booth in accordance with claim 1 or any one of claims 2 to 8, **characterised in that** the common ring-shaped collector duct (11) in the top view of the sun-tanning booth (1) is constructed ring-shaped and, with the door (5) closed, delimits the upper e.g. ring-shaped wall of the sun-tanning booth (1).

11. Upright sun-tanning booth in accordance with claim 1 or any one of claims 2 to 10, **characterised in that** the walls of the common ring-shaped collector duct (11) are rounded on the outside or are designed so that their form conforms to the outside contour of the outside (2) of the sun-tanning booth (1).

12. Upright sun-tanning booth in accordance with claim 1 or any one of claims 2 to 11, **characterised in that** the fan (14) conveys the exhaust air away through a square-shaped connecting hole (15) and that this connecting hole (15) forms part of a box-shaped projecting member (13) forming a single piece with the common ring-shaped collector duct (11) the inner flow cross section of which is constructed ring-shaped, polygonal or rectangular or square.

13. Upright sun-tanning booth in accordance with claim 1 or any one of claims 2 to 12, **characterised in that** in the base area of the interior space (3) at least one slot-shaped exhaust air hole (20) or preferably a number of slot-shaped or elongated exhaust air holes (20) are arranged at regular intervals around the circumference of the interior space (3) through which exhaust air holes (20) the air flows into the spaces in the door (5) in which the sun-tanning lamps are mounted and into the spaces in the fixed portion (9) of the sun-tanning booth (1).

14. Upright sun-tanning booth in accordance with claim 1 or any one of claims 2 to 13, **characterised in that** in the fixed portion (9) and in the door (5) sun-tanning lamps are disposed with their longitudinal axes parallel and spaced apart and the spaces in the fixed portion (9) and the door (5) in which the sun-tanning lamps are mounted are connected to the common ring-shaped collector duct (11) and in the area of the base (6) to the interior space (3) through one or more exhaust air holes (20).

15. Upright sun-tanning booth in accordance with claim 1 or any one of claims 2 to 14, **characterised in that** the fan (14) is connected through a suction nozzle, e.g. with the interposition of a vibration-damping bearing, to an upper, ring-shaped closing member (7) which incorporates in its inside the common ring-shaped collector duct (11).

16. Upright sun-tanning booth in accordance with claim 15, **characterised in that** the connecting nozzle is assigned a noise-damping element for the box-shaped projecting member (13).

17. Upright sun-tanning booth in accordance with claim 1 or any one of claims 2 to 16, **characterised in that** the sun-tanning lamps are grouped in circular ring sectors in the sandwich-like walls (16, 17) of the door (5) and those of the fixed portion (9) of the sun-tanning booth (1) and that each circular ring sector is connected to the respective ring sector through a slot-shaped exhaust air hole (20) leading into the interior space (3) in the area of the base (6) and that the individual ring sector ducts so formed lead on their upper side into the common ring-shaped collector duct (11) in the upper closing member (7).

## Revendications

1. Solarium vertical (1) avec porte (5) à ouvrir et fermer par rotation sur des axes verticaux, porte qui avec les parois fixes (18, 19) du solarium (1) délimite un volume intérieur (3), sachant que dans ledit volume intérieur (3) une personne debout peut prendre un bain de lumière grâce aux lampes de bronzage disposées dans les parois fixes (18, 19), avec au moins un ventilateur (14) entraîné par moteur et servant à faire pénétrer l'air de refroidissement et l'air frais dans le volume intérieur (3) pour qu'il entre en contact avec les lampes de bronzage et la personne, sachant qu'en haut, dans la zone de la tête, le volume intérieur (3) présente au moins un orifice d'afflux (12) servant à aspirer (E) l'air de refroidissement et l'air frais, cet air quittant ensuite le volume intérieur (3) par au moins un orifice d'évacuation (20) dans la zone d'un piètement d'appui (6), et traversant des cavités prévues dans les parois (18, 19) de la partie fixe (9) pour remonter (H et C) et refroidir les lampes de bronzage, **caractérisé en ce que** la porte (5) présente également des lampes de bronzage refroidies par l'air de refroidissement et l'air frais qui circulent vers le haut (H et C) par les cavités prévues dans les parois (16, 17) de la porte (5) ; et **en ce que** l'air de refroidissement et l'air frais quittent le solarium (1) via un conduit collecteur annulaire (11) conjoint agencés dans la zone de la tête, lequel conduit est raccordé soit à l'air extérieur, soit à un conduit d'air sortant.

2. Solarium vertical selon la revendication 1, **caractérisé en ce que** le volume intérieur (3) présente, en haut, dans la zone de la tête, un orifice d'afflux (12) centrique correspondant de préférence au diamètre intérieur (D) du volume intérieur (3) ou dimensionné plus petit que ledit diamètre, l'orifice d'afflux (12) étant limité axialement et par exemple aussi radialement par un corps terminal supérieur (7) qui présente un conduit collecteur annulaire (11) conjoint auquel a été affecté latéralement une soufflerie (14) sur le côté opposé au volume intérieur (3).

3. Solarium vertical selon la revendication 1 ou 2, **caractérisé en ce que** le ventilateur (14) configuré en soufflerie est une soufflerie radiale.

4. Solarium vertical selon la revendication 1 ou l'une des revendications 2 et 3, **caractérisé en ce que** la soufflerie (14) transporte l'air sortant via un orifice de raccordement (15) débouchant vers le haut dans le local, cet air soit débouchant dans l'air ambiant soit étant évacué via un conduit de raccordement ou un flexible hors de la pièce dans laquelle le solarium (1) a été installé.

5. Solarium vertical selon la revendication 1 ou l'une des revendications 2 à 4, **caractérisé en ce que** le conduit collecteur annulaire (11) conjoint, guidant ainsi l'air de refroidissement, relie entre elles, sur le côté frontal supérieur de la partie fixe (9) composant le solarium (1), toutes les cavités servant à refroidir les lampes de bronzage.

6. Solarium vertical selon la revendication 1 ou l'une des revendications 2 à 5, **caractérisé en ce qu'**au moteur d'entraînement de la soufflerie (14) est affecté un dispositif de commande ou de régulation servant à réguler ou piloter la vitesse.

7. Solarium vertical selon la revendication 1 ou l'une des revendications 2 à 6, **caractérisé en ce qu'**il est possible, au moyen d'un ou plusieurs capteurs thermométriques, de mesurer la température des lampes de bronzage, par exemple au niveau de leur surface ou dans le compartiment dans lequel se trouvent les lampes, et/ou de mesurer la température de l'air de refroidissement situé à l'extérieur du solarium (1), et qu'il est possible, via un dispositif de commande ou dispositif de régulation électronique, de piloter ou réguler en fonction de ces températures la vitesse du moteur entraînant la soufflerie (14).

8. Solarium vertical selon la revendication 7, **caractérisé en ce que** la température mesurée dans les compartiments dans lesquels se trouvent les lampes de bronzage et la température mesurée de l'air de refroidissement affluant (E) sont mesurables à l'aide d'au moins un capteur thermométrique agencé sur la peau de la personne à bronzer, **en ce qu'**il est possible d'entrer ces températures mesurées dans une mémoire électronique et de les comparer avec des valeurs déjà en mémoire et, en fonction de ces valeurs, de réguler ou piloter la vitesse du moteur d'entraînement donc le débit de refoulement de la soufflerie (14).

9. Solarium vertical selon la revendication 1 ou l'une des revendications 2 à 8, **caractérisé en ce que** le débit de refoulement de la soufflerie (14) est pilotable ou régulable en fonction de la puissance respectivement absorbée par les lampes de bronzage.

10. Solarium vertical selon la revendication 1 ou l'une des revendications 2 à 8, **caractérisé en ce que** conduit collecteur circulaire (11) conjoint présente, solarium (1) vu de dessus, une configuration annulaire circulaire et délimite, lorsque la porte (5) est fermée, la paroi supérieure par exemple annulaire du solarium (1).

11. Solarium vertical selon la revendication 1 ou l'une des revendications 2 à 10, **caractérisé en ce que** les parois du conduit collecteur circulaire (11) conjoint sont arrondies à l'extérieur et/ou configurées de sorte à s'adapter par leur forme au contour extérieur en présence sur le côté extérieur (2) du solarium (1).

12. Solarium vertical selon la revendication 1 ou l'une des revendications 2 à 11, **caractérisé en ce que** la soufflerie (14) refoule l'air sortant via un orifice de raccordement (15) de géométrie quadrangulaire carrée, et **en ce que** cet orifice de raccordement (15) fait partie d'une terminaison (13) en forme de caisson reliée monobloc avec le conduit collecteur annulaire (11) conjoint, conduit dont la section intérieure servant au passage du flux est configurée circulaire ou polygonale ou rectangulaire ou carrée.

13. Solarium vertical selon la revendication 1 ou l'une des revendications 2 à 12, **caractérisé en ce que** dans la zone de piètement du volume intérieur (3) sont agencés au moins un orifice d'air sortant (20) en forme de fente, de préférence plusieurs orifices d'air sortant (20) en forme de fentes ou étirés en longueur, disposés selon un certain espacement ainsi que répartis équidistants sur la périphérie du volume intérieur (3), orifices par lesquels l'air afflue dans les cavités recevant les lampes de bronzage, cavités ménagés dans la porte (5) et dans la partie fixe (9) du solarium (1).

14. Solarium vertical selon la revendication 1 ou l'une des revendications 2 à 13, **caractérisé en ce que** dans la partie fixe (9) et dans la porte (5) ont été agencées des lampes de bronzage parallèles selon leur axe longitudinal et espacées les unes des autres, lampes dont les cavités qui les reçoivent sont raccordées par un ou plusieurs orifices d'air sortant (20) au conduit collecteur circulaire (11) conjoint et d'autre part, dans la zone du piètement (6), au volume intérieur (3).

15. Solarium vertical selon la revendication 1 ou l'une des revendications 2 à 14, **caractérisé en ce que** la soufflerie (14) est raccordée via un embout d'aspiration, p. ex. avec intercalage d'un palier destiné à atténuer les vibrations, à un corps terminal (7) supérieur en forme d'anneau circulaire présentant, à l'intérieur du corps, le conduit collecteur annulaire (11) conjoint.

16. Solarium vertical selon la revendication 15, **caractérisé en ce qu'**à l'embout de raccordement de la terminaison (13) en forme de caisson est affecté un corps destiné à atténuer le bruit.

17. Solarium vertical selon la revendication 1 ou l'une des revendications 2 à 16, **caractérisé en ce que** les lampes de bronzage sont agencées réunies en secteurs circulaires de plusieurs lampes d'une part dans les parois (16, 17) de la porte (5) construites en sandwich et/ou d'autre part dans les parois (18, 19) de la partie fixe (9) du solarium (1), et **en ce que** chaque secteur annulaire est raccordé au secteur annulaire respectif via un orifice d'air sortant (20) en forme de fente dans la zone du piètement (6) et débouchant dans le volume intérieur (3), et **en ce que** les canaux de secteur annulaire individuels ainsi formés débouchent, sur leur côté supérieur, dans le conduit collecteur annulaire (11) conjoint du corps terminal (7) supérieur.
